# EUROPÄISCHE PATENTSCHRIFT

(11) **EP 2 461 160 B1**
(45) Veröffentlichungstag und Bekanntmachung des Hinweises auf die Patenterteilung: **12.06.2013**
(21) Anmeldenummer: 10015313.9
(22) Anmeldetag: 04.12.2010
(51) Int. Cl.: G01N 33/487, G01N 35/00

(54) **System zur Untersuchung einer Körperflüssigkeitsprobe**
System for analysing a bodily fluid sample
Système d'examen d'un échantillon de liquide corporel

(43) Veröffentlichungstag der Anmeldung: 06.06.2012
(73) Patentinhaber: Roche Diagnostics GmbH, 68305 Mannheim (DE); F. Hoffmann-La Roche AG, 4070 Basel (CH)
(72) Erfinder: Schwenker, Kai-Oliver, D-67454 Haßloch (DE); Lorenz, Robert, D-67547 Worms (DE)
(74) Vertreter: Twelmeier Mommer & Partner

(56) Entgegenhaltungen:
- EP-A1- 2 177 914
- WO-A1-2005/032372
- US-A- 4 954 319
- US-A1- 2002 188 224
- US-A1- 2007 119 710

## Beschreibung

Die Erfindung geht aus von einem System mit den im Oberbegriff des Anspruchs 1 angegebenen Merkmalen.

Derartige Systeme bieten einen hohen Benutzerkomfort, da auf einem Trägerband eine hohe Anzahl von Verbrauchselementen, beispielsweise Testfeldern zur Bestimmung der Glucosekonzentration, angeordnet werden kann. Bei einem Messgerät muss deshalb der für viele Benutzer lästige Austausch einer Bandkassette nur selten vorgenommen werden.

An solchen Bandkassetten kann ein wiederbeschreibarer Datenträger, beispielsweise ein RFID, angebracht sein, auf dem die Anzahl der noch unbenutzten Verbrauchselemente gespeichert ist. Diese Anzahl kann dann von einem Messgerät, in das die Kassette eingesetzt ist, jedes mal aktualisiert werden, wenn ein Verbrauchselement verwendet wird. Auf diese Weise kann der noch in einer Kassette vorhandene Vorrat an Verbrauchselementen zuverlässig überwacht und einem Benutzer angezeigt werden, wenn der Vorrat zur Neige geht. Nachteilig hieran sind die mit dem Datenträger verbundenen Kosten.

US 2002/188224 offenbart ebenfalls ein System mit einem Trägerband das mehrere Verbrauchselemente trägt, mit einer Anzeige über die größe des Vorrats an noch unbenutzten Verbrauchselementen.

Aufgabe der vorliegenden Erfindung ist es deshalb, einen kostengünstigeren Weg aufzuzeigen, wie eine Information über die Größe des Vorrats an noch unbenutzten Verbrauchselementen eines Trägerbandes gewonnen werden kann, so dass ein Benutzer nicht von der Notwendigkeit eines Wechsels einer Bandkassette überrascht wird.

Diese Aufgabe wird durch ein System mit den im Anspruch angegebenen Merkmalen sowie durch ein Verfahren gemäß Anspruch 15 gelöst. Vorteilhafte Weiterbildungen der Erfindung sind Gegenstand von Unteransprüchen.

Bei einem erfindungsgemäßen System werden aus einem Drehwinkel und einer Bandtransportlänge, um die das Trägerband bei einer Drehung des Wickels um diesen Drehwinkel bewegt wird, die Information über die Größe des Vorrats an noch unbenutzten Verbrauchselementen des Trägerbandes ermittelt. Dabei kann erfasst werden, wie weit sich der Wickel drehen muss, d.h. um welchen Winkel, damit das Trägerband um eine vorgegebene Transportlänge weiterbewegt wird. Die vorgegebene Transportlänge kann beispielsweise dem Abstand zwischen zwei Verbrauchselementen oder dem Abstand zwischen an dem Trägerband angebrachten Markierungen entsprechen, die beim Bandtransport an einem Sensor vorbeigeführt werden.

Die durch eine volle Umdrehung des Wickels bewirkte Transportbewegung des Trägerbandes ist nämlich umso größer, je größer die Stärke des Wickels ist. Bei einer Drehung um einen gegebenen Winkel wird also das Trägerband um eine umso größere Bandtransportlänge weiter bewegt, je größer der Durchmesser des Wickels ist. Da die Stärke des Wickels umso größer ist, je größer die bereits aufgewickelte Bandlänge ist, kann erfindungsgemäß durch Auswertung des mit einer Bandtransportlänge verbundenen Drehwinkels eine Information über die bereits aufgewickelte Bandlänge und damit über die Anzahl der bereits benutzen Verbrauchselemente gewonnen werden. Die von der Wickeleinrichtung bereits aufgewickelte Bandlänge ist nämlich ein Maß für die Anzahl der bereits benutzten Verbrauchselemente. In entsprechender Weise ist die verbleibende Bandlänge proportional zur Anzahl der noch zur Verfügung stehenden Verbrauchselemente.

Wenn die Stärke des Wickels, insbesondere dessen Durchmesser, eine kritische Grenze überschreitet, bedeutet dies deshalb, dass der Vorrat an noch unbenutzten Verbrauchselementen bald erschöpft ist. Anstatt direkt die Stärke des Wickels zu messen oder fortlaufend die Längen der jeweils bei Betätigen der Wickeleinrichtung aufgewickelten Bandabschnitte aufzuaddieren, wird erfindungsgemäß aus dem mit einem Drehwinkel verbunden Bandvorlauf auf die Stärke des Wickels geschlossen und so eine Information über die Größe des Vorrats an noch unbenutzten Verbrauchselementen des Trägerbandes gewonnen. Der sich bei einem gegebenen Drehwinkel ergebende Bandvorlauf ist nämlich ein Maß für den bereits verbrauchten Anteil eines von einem Trägerband zur Verfügung gestellten Vorrats an Verbrauchselementen.

Aus dem Drehwinkel und der Bandtransportlänge, um die das Trägerband bei einer Drehung des Wickels um diesen Drehwinkel bewegt wird, kann die genaue Anzahl der bereits benutzten Verbrauchselemente ermittelt werden. Ist die Anzahl der von einem Trägerband getragenen Verbrauchselemente bekannt, was in der Regel der Fall ist, so kann damit auch die Anzahl der noch unbenutzten Verbrauchselemente berechnet werden. Um einen Benutzer rechtzeitig darauf hinzuweisen, dass der Vorrat an Verbrauchselementen bald zu Neige geht, ist es jedoch nicht unbedingt erforderlich die Anzahl der noch nicht verbrauchten Verbrauchselemente präzise zu ermitteln, sondern es genügt in der Regel eine ungefähre Angabe, beispielsweise mit einer Ungenauigkeit von bis zu 3 Verbrauchselementen oder bis zu 10% bezogen auf die Gesamtzahl der Verbrauchselemente eine frischen Trägerbands.

Einem Benutzer kann eine Information über die Größe des Vorrats an unbenutzten Verbrauchselementen des Trägerbandes beispielsweise auch angezeigt werden, indem ein Warnsignal, beispielsweise ein Leuchtsignal, erzeug wird, sobald die Stärke des Wickels einen kritischen Schwellenwert übersteigt. Möglich ist es insbesondere auch, die Größe des Vorrats an noch unbenutzten Verbrauchselementen des Trägerbandes als einen Balken anzuzeigen, dessen Länge mit der Anzahl der noch unbenutzten Verbrauchselemente korreliert, also beispielsweise umso kürzer ist, je weniger unbenutzte Verbrauchselemente noch vorhanden sind.

Eine vorteilhafte Weiterbildung der Erfindung sieht vor, dass das Trägerband in regelmäßigen Abständen angeordnete Markierungen aufweist und die Markierungen zur Messung der Bandtransportlänge, also des Bandvorlaufs, von einem Sensor erfasst werden. Besonders gut geeignet sind optische Sensoren, beispielsweise Fotodioden, Fototransistoren oder andere lichtsensitive Sensoren.

Um Markierungen zu detektieren, kann man mit einer Lichtquelle einen Messfleck auf dem Trägerband beleuchten und diesen Messfleck mit einem Sensor überwachen. Wenn sich eine Markierung durch den Messfleck bewegt, ändert sich die von dem Sensor erfasste Helligkeit, so dass die Markierung detektiert werden kann.

Die Markierungen können im einfachsten Fall als Durchbrüche oder Aussparungen des Trägerbands ausgebildet sein, wobei Lichtquelle und Sensor auf verschiedenen Seiten des Trägerbands angeordnet sind. Die Detektion einer Markierung funktioniert dann nach dem Prinzip einer Lichtschranke. Bevorzug ist es aber, die Markierungen durch Reflektion zu detektieren. Dafür ist es günstig, wenn sich die Markierungen in ihrer Reflektivität möglichst deutlich von den übrigen Bereichen des Trägerbandes unterscheiden, also einen möglichst deutlichen Kontrast bilden. Helle Flächen oder Metallbeschichtungen haben eine hohe Reflektivität und damit einen hohen Kontrast zu dunklen, insbesondere schwarzen Bereichen. Der Begriff Reflektion wird hier in seiner allgemeinen Bedeutung verwendet, so dass davon auch die Remission, d.h. ungerichtete oder diffuse Reflektionen, ebenso wie die Remission von Licht anderer Wellenlänge umfasst ist. Beispielsweise können für die Positionsmarken auch fluoreszierende Pigmente oder Fluoreszenzfarbstoffe angesetzt werden.

Besonders vorteilhaft können auf dem Trägerband Stoppmarkierungen vorgesehen sein, deren Erfassung durch den Sensor ein Stoppen der Wickeleinrichtung bewirkt, so dass ein Verbrauchselement in einer Gebrauchsposition positioniert wird, beispielsweise kann ein Testfeld in eine Position für eine Probenaufnahme gebracht werden. Weitere Markierungen können auf dem Trägerband vorgesehen sein, um auch zwischen zwei Stoppmarkierungen eine einfache Messung der Bandlaufgeschwindigkeit bzw. einer Bandtransportlänge zu ermöglichen.

Eine weitere vorteilhafte Weiterbildung der Erfindung sieht vor, dass die Wickeleinrichtung in Abhängigkeit von der ermittelten Information über die Größe des Vorrats an noch unbenutzten Verbrauchselementen des Trägerbandes gesteuert wird. Indem die Information über die Größe des Vorrats an noch unbenutzten Verbrauchselementen einer Steuereinrichtung zur Verfügung gestellt wird, kann die Rotationsgeschwindigkeit der Wickeleinrichtung zum Bandtransport an die Stärke des Wickels angepasst werden. Auf diese Weise lässt sich erreichen, dass die zum Bereitstellen eines weiteren Verbrauchselements erforderliche Zeit weitestgehend unabhängig von der Wickelstärke ist. Unter Verwendung der ermittelten Informationen über die Größe des Vorrats an noch unbenutzten Verbrauchselementen kann also die Wickeleinrichtung so gesteuert werden, dass für die Zeit zum Bereitstellen eines weiteren Verbrauchselements ein Sollwert vorgegeben wird.

Beispielsweise kann beim Bereitstellen eines Verbrauchselements eine Bandlaufgeschwindigkeit auf eine von der Stärke des Wickels unabhängigen Sollwert geregelt werden. Unter der Bandlaufgeschwindigkeit beim Bereitstellen eines Verbrauchselements ist dabei eine mittlere Bandlaufgeschwindigkeit zu verstehen, da das Trägerband zu Beginn einer Transportbewegung zunächst beschleunigt und anschließend wieder abgebremst wird. Die Bandlaufgeschwindigkeit beim Bereitstellen eines Verbrauchselements ist der Quotient aus dem Abstand zwischen zwei Verbrauchselementen und der zum Transport des Bandes über die entsprechende Weglänge benötigten Zeit.

Manche Benutzer könnten es als irritierend empfinden, dass die zum Bereitstellen eines Verbrauchselements erforderliche Zeit in Abhängigkeit von der Stärke des Wickels starken Schwankungen unterliegt. Hier lässt sich Abhilfe schaffen, indem man dem System eine Steuereinrichtung hinzufügt, um die Bandlaufgeschwindigkeit auf einen von der Stärke des Wickels unabhängigen Sollwert zu regeln. Für eine solche Regelung ist es nicht unbedingt erforderlich, dass eine Information über die Größe des Vorrats an noch unbenutzten Verbrauchselementen des Trägerbandes ermittelt und angezeigt wird. Es genügt beispielsweise, die momentane Bandtransportgeschwindigkeit mittels an dem Trägerband angebrachten Markierungen laufend zu überwachen und soweit erforderlich die Rotationsgeschwindigkeit des Wickels zu erhöhen oder zu senken, damit ein Verbrauchselement in der angestrebten Sollzeit bereitgestellt wird, also in seine Gebrauchsposition, beispielsweise für eine Probenaufnahme oder eine Konzentrationsmessung, gelangt. Ein Aspekt der Erfindung, der auch selbstständige Bedeutung haben kann betrifft deshalb ein System zur Untersuchung einer Körperflüssigkeitsprobe, mit einem Trägerband, das mehrere Verbrauchselemente trägt, wobei ein Ende des Trägerbandes an einer Winkeleinrichtung befestigt ist, um zum Bandtransport das Trägerband zusammen mit benutzten Verbrauchselementen zu einem Wickel aufzuwickeln, und mit einer Steuereinrichtung, um die Bandlaufgeschwindigkeit auf einen von der Stärke des Wickels unabhängigen Sollwert zu regeln.

Die ermittelte Information über die Größe des Vorrats an noch unbenutzten Verbrauchselementen des Trägerbandes kann in einem Speicher abgelegt werden. Falls die Wickeleinrichtung so gesteuert werden soll, dass die Zeit für das Bereitstellen eines weiteren Verbrauchselements konstant ist, kann die Wickeleinrichtung bei Betätigung sofort unter Berücksichtigung der entsprechenden Information angesteuert werden. Bei Gebrauch eines Verbrauchselements kann der gespeicherte Zählerstand aktualisiert, also um eins geändert werden, beispielsweise kann der Zählerstand die Anzahl der noch benutzbaren Verbrauchselemente angeben und jeweils um eins dekrementiert werden.

Eine weitere vorteilhafte Weiterbildung der Erfindung sieht vor, dass die Bandtransportlänge in Abhängigkeit von der Drehwinkelstellung des Wickels erfasst, daraus ein Wert für die Unwucht des Wickels ermittelt wird und anhand von diesem Wert ein frisches Trägerband, das nur unbenutzte Verbrauchselemente trägt, von einem bereits teilweise aufgewickelten Trägerband, das wenigstens ein benutztes Verbrauchselement trägt, unterschieden wird. Testfelder und andere Verbrauchselemente, die sich in Längsrichtung des Trägerbands erstrecken und weniger flexibel als das Trägerband sind, können beim Aufwickeln eine erhebliche Unwucht des Wickels verursachen.

Diese Unwucht macht sich darin bemerkbar, dass die Bandtransportlänge nicht nur von dem Drehwinkel des Wickels abhängt, sondern zusätzlich auch von dessen Drehwinkelstellung. Bei einem frischen Trägerband sind in der Regel noch keine Verbrauchselemente auf die Wickeleinrichtung aufgewickelt, so dass keine Unwucht vorhanden ist. An dem Fehlen einer Unwucht kann deshalb ein frisches Trägerband erkannt werden. Auf diese Weise kann ein erfindungsgemäßes System automatisch erkennen, wenn eine neue Bandkassette, die ein frisches Trägerband enthält, in ein Messgerät eingelegt wird. Ein für die Unwucht des Wickels ermittelter Wert kann in einem Speicher abgelegt werden. Wird später ein geänderter Wert für die Unwucht festgestellt, kann daraus auf den Austausch eines Trägerbands geschlossen werden. Dabei kann sogar der Fall erfasst werden, dass ein verbrauchtes oder teilweise verbrauchtes Trägerband gegen ein Trägerband ausgetauscht, das bereits teilweise aufgewickelt ist, also auch bereits benutzte Verbrauchselemente trägt.

Bei den Verbrauchselementen handelt es sich bevorzugt um Testelemente zur Messung einer Analytkonzentration in einer Körperflüssigkeitsprobe. Derartige Testelemente enthalten in der Regel Nachweisreagenzien, die bei Kontakt mit einer Körperflüssigkeitsprobe eine Nachweisreaktion bewirken. Gebräuchlich sind insbesondere Nachweisreaktionen zur fotometrischen oder elektrochemischen Bestimmung einer Analytkonzentration, beispielsweise der Glucosekonzentration oder der Laktatkonzentration. Bei den Verbrauchselementen kann es sich beispielsweise auch um Stechelemente handeln. Möglich sind dabei sowohl Trägerbänder, die Stechelemente und zwischen den Stechelementen angeordnete Testelemente tragen als auch Trägerbänder, die Stechelemente mit integrierten Testelementen tragen, sowie Trägerbänder, die ausschließlich Stechelemente tragen.

Weitere Einzelheiten und Vorteile der Erfindung werden an einem Ausführungsbeispiel unter Bezugnahme auf die beigefügten Zeichnungen erläutert. Es zeigen:
- Fig. 1: eine schematische Darstellung eines Systems zur Untersuchung einer Körperflüssigkeitsprobe;
- Fig. 2: eine Explosionsdarstellung eines Ausführungsbeispiels einer Bandkassette mit einem Verbrauchselemente tragenden Trägerband; und
- Fig. 3: eine schematische Darstellung eines Ausführungsbeispiels eines Trägerbands mit Verbrauchselementen und Markierungen.

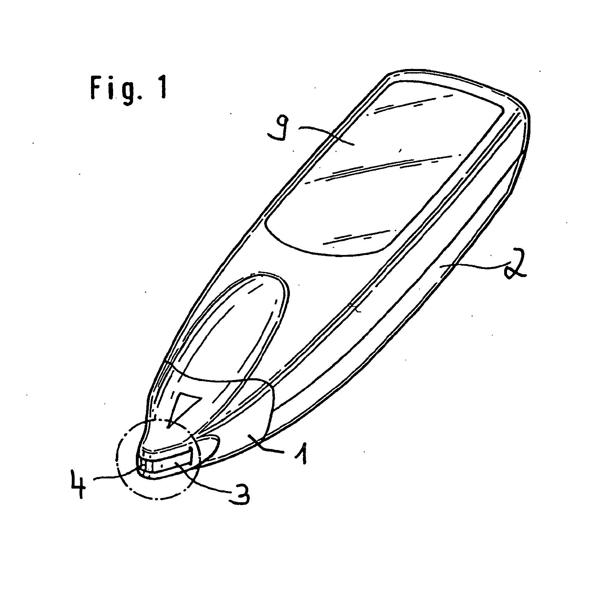

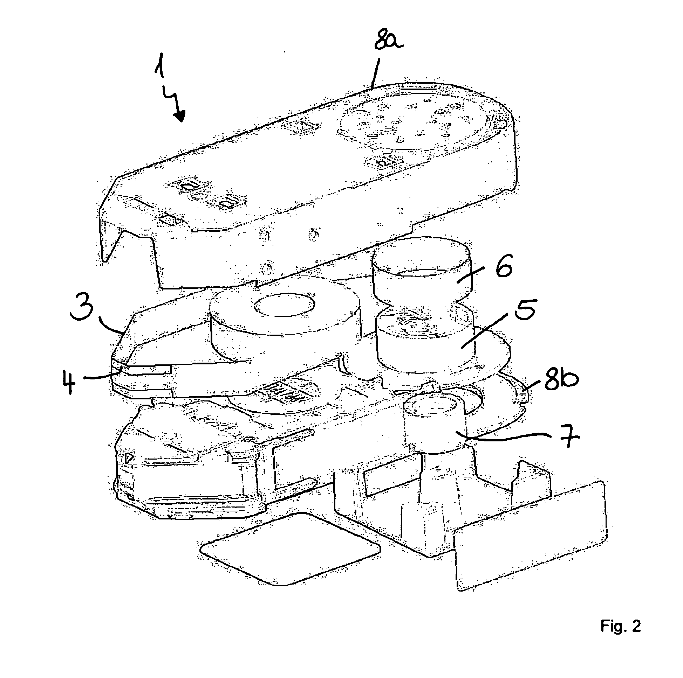

Figur 1 zeigt ein System zur Messung einer Analytkonzentration einer Körperflüssigkeitsprobe, beispielsweise zur Messung der Glucosekonzentration von Blut und/oder interstitieller Flüssigkeit. Derartige Systeme werden beispielsweise von Diabetikern benötigt, die mehrmals täglich ihren Blutzuckergehalt messen müssen. Das dargestellte System 1 umfasst als erste Systemkomponente eine in Figur 2 dargestellte Bandkassette 1 und als weitere Systemkomponente ein Handgerät 2. In Figur 1 ist das Handgerät 2 mit einer eingesetzten Bandkassette 1 dargestellt.

Die in Figur 2 dargestellte Bandkassette 1 enthält ein Trägerband 3, das Verbrauchselemente 4 in Form von Testelementen trägt. Die Testelemente können beispielsweise als Testfelder mit Nachweisreagenzien ausgebildet sein, die bei Kontakt mit einer Körperflüssigkeitsprobe eine Nachweisreaktion bewirken, beispielsweise eine fotometrisch auswertbare Verfärbung in Abhängigkeit von einer Analytkonzentration. In Figur 2 ist wegen der besseren Übersichtlichkeit nur ein einziges Verbrauchselement 4 dargestellt. Tatsächlich trägt das Trägerband 3 aber eine größere Zahl von Testelementen, so dass mit dem Trägerband beispielsweise bis zu 50 oder noch mehr Konzentrationsbestimmungen durchgeführt werden können.

Ein Ende des in der Bandkassette 1 enthaltenen Trägerbands 3 ist an einer Wickeleinrichtung 5, nämlich einer Wickelrolle oder einem Dorn befestigt. Zum Bandtransport kann das Trägerband 3 zusammen mit benutzten Verbrauchselementen 4 zu einem Wickel 6 auf die Wickeleinrichtung 5 aufgewickelt werden. Ein Abschnitt des Trägerbandes 3 mit unbenutzten Verbrauchselementen ist von einem Gehäuse 8a, 8b der Bandkassette 1 umgeben und auf einer Rolle 7 als Vorratswickel aufgewickelt.

Das in Figur 1 dargestellte Handgerät 2 hat eine elektrische Anzeige 9 um Messergebnisse anzuzeigen. Mit der Anzeige 9 kann auch eine Information über die Größe des Vorrats an noch unbenutzten Verbrauchselementen 4 des Trägerbands 3 einer in das Handgerät 2 eingesetzten Kassette 1 angezeigt werden. Beispielsweise kann die Anzahl der noch unbenutzten Verbrauchselemente 4 angezeigt werden oder ein Warnsignal angezeigt werden, wenn der Vorrat eine kritische Schwelle unterschreitet.

Die Information über die Größe des Vorrats an noch unbenutzten Verbrauchselementen 4 wird bei dem dargestellten System aus einem Drehwinkel und einer Bandtransportlänge, um die das Trägerband 3 bei einer Drehung des Wickels 6 um diesen Drehwinkel vorwärts bewegt wird, ermittelt. Je größer nämlich der Wickel 6 ist, desto weiter wird das Trägerband 3 bei einer Drehung des Wickels 6 um einen gegebenen Winkel transportiert. Da die Stärke des Wickels 6 umso größer ist, je mehr Verbrauchselemente 4 bereits benutzt wurden, kann aus dem Drehwinkel und einem damit bewirkten Bandvorlauf, d. h. einer Bandtransportlänge, eine Information über die Stärke des Wickels b und damit auch über die Größe des Vorrats an noch unbenutzten Verbrauchselementen 4 des Trägerbandes 3 ermittelt werden.

Das Handgerät 2 enthält eine nicht dargestellte Auswerteeinheit, die dafür eingerichtet ist, aus einem Drehwinkel und einer Bandtransportlänge, um die das Trägerband 3 bei einer Drehung des Wickels 6 um diesen Drehwinkel bewegt wird, die Information über die Größe des Vorrats an noch unbenutzten Verbrauchselementen 4 des Trägerbands 3 zu ermitteln. Den Drehwinkel kann die Auswerteeinheit beispielsweise von einem Elektromotor erhalten, der zum Antreiben der Wickeleinrichtung 5 vorgesehen ist. Möglich ist es auch, einen zusätzlichen Drehwinkelsensor vorzusehen, welcher den Drehwinkel der Wickeleinrichtung 5 oder einer die Wickeleinrichtung antreibenden Welle detektiert.

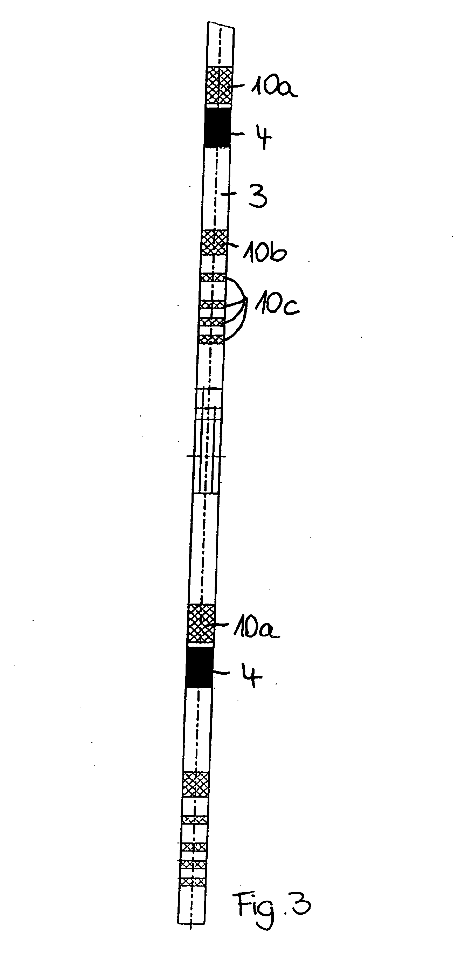

Wie insbesondere Figur 3 zeigt, trägt das Trägerband 3 verschiedene Markierungen 10a, 10b, 10c, die zur Messung der Bandtransportlänge von einem nicht dargestellten Sensor des Handgeräts 2 erfasst werden können. Die Markierungen 10a, 10b, 10c können beispielsweise als schwarze Streifen ähnlich wie ein Barcode auf dem Trägerband 3 angebracht sein.

Bei dem in Figur 3 dargestellten Ausführungsbeispiel bilden die Markierungen 10a Stoppmarkierungen, deren Erfassung durch einen nicht dargestellten Sensor des Handgeräts 2 ein Stoppen der Wickeleinrichtung 5 bewirkt. Zusätzlich sind weitere Markierungen 10b, 10c vorgesehen. Bevorzugt ist eine größere Anzahl von Markierungen 10c in konstanten Abständen zwischen zwei Testfeldern 4 angeordnet. Mit diesen Markierungen 10c kann beispielsweise die Bandlaufgeschwindigkeit gemessen werden. In der schematischen Darstellung von Figur 3 ist nur ein Teil der Markierungen 10c eingezeichnet. Tatsächlich weist das Trägerband 3 zwischen zwei Verbrauchselementen eine wesentlich größere Anzahl von Markierungen auf

Zusätzlich ist bei dem dargestellten Ausführungsbeispiel eine Markierung 10b vorgesehen, die einer Serie von Markierungen 10c vorangeht und beispielsweise eine Aktivierung einer nicht dargestellten Messeinrichtung des Handgeräts 2 für eine bald bevorstehende Messung bewirken kann.

Die Wickeleinrichtung 5 wird bevorzugt von einem Elektromotor angetrieben. Die Rotationsgeschwindigkeit der Wickeleinrichtung 5 zum Bandtransport wird bei abnehmender Anzahl der noch unbenutzten Verbrauchselemente 4 des Trägerbandes 3 reduziert. Dies bedeutet, dass die Wickeleinrichtung 5 in Abhängigkeit von der ermittelten Information über die Größe des Vorrats an noch unbenutzten Verbrauchselementen 4 des Trägerbandes gesteuert wird. Auf diese Weise lässt sich erreichen, dass die zum Bereitstellen eines Verbrauchselements 4 erforderliche Zeit unabhängig von der Stärke des Wickels 6 konstant ist.

Die aus dem Drehwinkel und einer dazu gehörenden Bandtransportlänge, um die das Trägerband 3 bei einer Drehung des Wickels 6 um diesen Drehwinkel bewegt wird, gewonnene Information über die Größe Vorrats an noch unbenutzten Verbrauchselementen 4 des Trägerbandes 3 kann in einem Zähler abgespeichert werden. Bei Gebrauch eines Verbrauchselements 4 wird der Zählerstand dann um eins geändert. Beim Betätigen der Wickeleinrichtung 5 kann eine nicht dargestellte Steuereinheit dann diese Information als wahrscheinlich richtig annehmen und gegebenenfalls auf der Grundlage einer Bandlaufgeschwindigkeitsmessung korrigieren.

Ein Testelement 4, wie es in Figur 2 dargestellt ist, hat in der Regel eine geringere Biegsamkeit als das Trägerband 3. Beim Aufwickeln kann ein benutztes Verbrauchselement 4 deshalb zu einer Unwucht des Wickels 6 führen. Eine solche Unwucht des Wickels 6 führt dazu, dass die Bandtransportlänge nicht nur von der Größe eines Drehwinkels, sondern auch von der Drehwinkelstellung des Wickels 6 abhängt. Indem die Bandtransportlänge in Abhängigkeit von der Drehwinkelstellung des Wickels 6 erfasst wird, kann deshalb ein Wert für die Unwucht des Wickels 6 ermittelt werden. Anhand von diesem Wert kann ein frisches Trägerband 3, das unbenutzte Verbrauchselemente 4 trägt, von einem bereits teilsweise aufgewickelten Trägerband 3, das wenigstens ein benutztes Verbrauchselement 4 trägt, unterschieden werden. Vorteilhaft kann auf diese Weise der Austausch einer Bandkassette 1 von dem Handgerät 2 erkannt und bei der Ansteuerung der Wickeleinrichtung 5 berücksichtigt werden.

### Bezugszahlen

- 1: Bandkassette
- 2: Handgerät
- 3: Trägerband
- 4: Verbrauchselement
- 5: Wickeleinrichtung
- 6: Wickel
- 7: Rolle
- 8a: Gehäuse
- 8b: Gehäuse
- 10a: Markierung
- 10b: Markierung
- 10c: Markierung

## Patentansprüche

1. System zur Untersuchung einer Körperflüssigkeitsprobe,
mit einem Trägerband (3), das mehrere Verbrauchselemente (4) trägt, wobei ein Ende des Trägerbandes (3) an einer Wickeleinrichtung (5) befestigt ist, um zum Bandtransport das Trägerband (3) zusammen mit benutzten Verbrauchselementen (4) zu einem Wickel (6) aufzuwickeln, und
mit einer Anzeige, um eine Information über die Grösse des Vorrats an noch unbenutzten Verbrauchselementen (4) des Trägerbandes (3) anzuzeigen,
**dadurch gekennzeichnet, dass**
das System dazu eingerichtet ist einen Drehwinkel und eine Bandtransportlänge, um die das Trägerband (3) bei einer Drehung des Wickels (6) um diesen Drehwinkel bewegt wird, zu ermitteln, und mit Hilfe dessen die Information über die Grösse des Vorrats an noch unbenutzten Verbrauchselementen (4) des Trägerbandes (3) zu ermitteln.

2. System nach Anspruch 1, **dadurch gekennzeichnet, dass** das Trägerband (3) in regelmäßigen Abstanden angeordnete Markierungen (10a, 10b, 10c) aufweist und die Markierungen (10a, 10b, 10c) zur Messung der Bandtransportlänge von einem Sensor, vorzugsweise einem optischen Sensor, erfasst werden.

3. System nach Anspruch 2, **dadurch gekennzeichnet, dass** das Trägerband (3) Stopp-Markierungen (10a), deren Erfassung durch den Sensor ein Stoppen der Wickeleinrichtung (5) bewirkt, und weitere Markierungen (10b, 10c) trägt.

4. System nach einem der Ansprüche 2 bis 3, **gekennzeichnet durch** eine Lichtquelle zum Beleuchten eines Messflecks auf dem Trägerband (3), der von dem Sensor erfasst wird.

5. System nach einem der vorstehenden Ansprüche, **gekennzeichnet durch** einen Elektromotor zum Antreiben der Wickeleinrichtung (5).

6. System nach einem der vorstehenden Ansprüche, **dadurch gekennzeichnet, dass** die Wickeleinrichtung (5) in Abhängigkeit von der ermittelten Information über die Größe des Vorrats an noch unbenutzten Verbrauchselementen (4) des Trägerbandes (3) gesteuert wird.

7. System nach einem der vorstehenden Ansprüche, **dadurch gekennzeichnet, dass** die Verbrauchselemente (4) Testelemente und/oder Lanzetten sind.

8. System nach einem der vorstehenden Ansprüche, **dadurch gekennzeichnet, dass** die Anzeige eine elektrische Anzeige ist.

9. System nach einem der vorstehenden Ansprüche, **dadurch gekennzeichnet, dass** als die Information über die Größe des Vorrats an noch unbenutzten Verbrauchselementen (4) des Trägerbandes (3) die Anzahl der noch unbenutzten Verbrauchselemente (4) ermittelt wird.

10. System nach einem der vorstehenden Ansprüche, **dadurch gekennzeichnet, dass** die Rotationsgeschwindigkeit der Wickeleinrichtung (5) zum Bandtransport mit abnehmender Anzahl der noch unbenutzten Verbrauchselemente (4) des Trägerbandes (3) reduziert wird.

11. System nach einem der vorstehenden Ansprüche, **gekennzeichnet durch** eine Steuereinrichtung, um beim Bereitstellen eines Verbrauchselements (4) eine Bandlaufgeschwindigkeit auf einen von der Stärke des Wickels (6) unabhängigen Sollwert zu regeln.

12. System nach einem der vorstehenden Ansprüche, **dadurch gekennzeichnet, dass** die Bandtransportlänge in Abhängigkeit von der Drehwinkelstellung des Wickels (6) erfasst, daraus ein Wert für die Unwucht des Wickels (6) ermittelt wird, und anhand von diesem Wert ein frisches Trägerband (3), das nur unbenutzte Verbrauchselemente (4) trägt, von einem bereits teilweise aufgewickelten Trägerband (3), das wenigstens ein benutztes Verbrauchselement (4) trägt, unterschieden wird.

13. System nach einem der vorstehenden Ansprüche, **dadurch gekennzeichnet, dass** bei Gebrauch eines Verbrauchselements (4) ein Zählerstand um eins geändert wird.

14. System nach einem der vorstehenden Ansprüche, **dadurch gekennzeichnet, dass** das Trägerband (3) in einer Bandkassette (1) enthalten ist.

15. Verfahren zum Überwachen eines noch verbleibenden Vorrats von unbenutzten Verbrauchselementen (4), die von einem Trägerband (3) getragenen werden, das bei Gebrauch zusammen mit bereits benutzten Verbrauchselementen (4) zu einem Wickel (6) aufgewickelt wird, **dadurch gekennzeichnet, dass** aus einem Drehwinkel und einer Bandtransportlänge, um die das Trägerband (3) bei einer Drehung des Wickels (6) um diesen Drehwinkel bewegt wird, die Information über die Größe des Vorrats an noch unbenutzten Verbrauchselementen (4) des Trägerbandes (3) ermittelt wird.

## Claims

1. A system for testing a sample of bodily fluid,
comprising a carrier band (3), which carries a plurality of consumable elements (4), wherein one end of the carrier band (3) is attached to a wind-up device (5) for winding the carrier band (3), together with used consumable elements (4), onto a reel (6) for band transport, and
comprising a display for displaying information regarding the supply of yet unused consumable elements (4) of the carrier band (3),
**characterized in that**
the system is adapted for detecting an angle of rotation and a band transport length by which the carrier band (3) is moved with a rotation of the reel (6) about said angle of rotation, and to determine by means of this angle the information regarding the supply of yet unused consumable elements (4) of the carrier band (3).

2. The system according to claim 1, **characterized in that** the carrier band (3) has markings (10a, 10b, 10c) disposed at regular intervals, and the markings (10a, 10b, 10c) are detected by a sensor, preferably an optical sensor, for the purpose of measuring the band transport length.

3. The system according to claim 2, **characterized in that** the carrier band (3) carries stop markings (10a), the detection of which by the sensor causes the wind-up device (5) to stop, along with other markings (10b, 10c).

4. The system according to one of claims 2 to 3, **characterized by** a light source for illuminating a measuring spot on the carrier band (3), which spot is detected by the sensor.

5. The system according any one of the preceding claims, **characterized by** an electric motor for driving the wind-up device (5).

6. The system according to any one of the preceding claims, **characterized in that** the wind-up device (5) is controlled on the basis of the obtained information regarding the supply of unused consumable elements (4) of the carrier band (3).

7. The system according to any one of the preceding claims, **characterized in that** the consumable elements (4) are test elements and/or lancets.

8. The system according to any one of the preceding claims, **characterized in that** the display is an electrical display.

9. The system according to any one of the preceding claims, **characterized in that** the information regarding the supply of unused consumable elements (4) is determined as the number of unused consumable elements (4) of the carrier band (3).

10. The system according to any one of the preceding claims, **characterized in that** the rotational speed of the wind-up device (5) for band transport is reduced as the number of unused consumable elements (4) of the carrier band (3) decreases.

11. The system according to any one of the preceding claims, **characterized by** a control device for controlling a band travel speed, while a consumable element (4) is being provided, to a target value that is independent of the thickness of the reel (6).

12. The system according to any one of the preceding claims, **characterized in that** the band transport length is detected on the basis of the rotational angle position of the reel (6), from which a value for the imbalance of the reel (6) is determined, and, on the basis of this value, a fresh carrier band (3), which carries only unused consumable elements (4), is distinguished from a carrier band (3) which has already been partially wound up, and which carries at least one used consumable element (4).

13. The system according to any one of the preceding claims, **characterized in that** when a consumable element (4) is used, a counter status is adjusted by one.

14. The system according to any one of the preceding claims, **characterized in that** the carrier band (3) is contained in a band cartridge (1).

15. A method for monitoring a supply of unused consumable elements (4), which are carried by a carrier band (3) and which, when used, are wound, together with consumable elements (4) that have already been used, onto a reel (6), c**haracterized in that** an information regarding the supply of unused consumable elements (4) of the carrier band (3) is obtained from an angle of rotation and from a band transport length by which the carrier band (3) is moved with a rotation of the reel (6) about said angle of rotation.

## Revendications

1. Système d'examen d'un échantillon de liquide corporel,
comprenant une bande de support (3) qui porte plusieurs éléments consommables (4), une extrémité de la bande de support (3) étant fixée sur un dispositif d'enroulement (5) pour enrouler la bande de support (3) avec les éléments consommables utilisés (4) et en former une bobine (6) pour le transport de la bande, et
comprenant un affichage pour afficher une information sur la quantité d'éléments consommables (4) non encore utilisés en réserve sur la bande de support (3),
**caractérisé en ce que** le système est réglé pour déterminer un angle de rotation et une longueur de transport de bande sur laquelle la bande de support (3) est déplacée lors d'une rotation de la bobine (6) sur cet angle de rotation et pour déterminer, à l'aide de ces éléments, l'information sur la quantité d'éléments consommables (4) non encore utilisés en réserve sur la bande de support (3).

2. Système selon la revendication 1, **caractérisé en ce que** la bande de support (3) est pourvue de repères (10a, 10b, 10c) disposés à intervalles réguliers et les repères (10a, 10b, 10c) sont détectés par un capteur, de préférence un capteur optique, pour mesurer la longueur de transport de bande.

3. Système selon la revendication 2, **caractérisé en ce que** la bande de support (3) porte des repères d'arrêt (10a) dont la détection par le capteur entraîne un arrêt du dispositif d'enroulement (5) et d'autres repères (10b, 10c).

4. Système selon l'une des revendications 2 à 3, **caractérisé par** une source lumineuse destinée à l'éclairage d'un point de mesure sur la bande de support (3) qui est détecté par le capteur.

5. Système selon l'une des revendications précédentes, **caractérisé par** un moteur électrique pour entraîner le dispositif d'enroulement (5).

6. Système selon l'une des revendications précédentes, **caractérisé en ce que** le dispositif d'enroulement (5) est commandé en fonction de l'information déterminée sur la quantité d'éléments consommables (4) non encore utilisés en réserve sur la bande de support (3).

7. Système selon l'une des revendications précédentes, **caractérisé en ce que** les éléments consommables (4) sont des éléments d'analyse et/ou des lancettes.

8. Système selon l'une des revendications précédentes, **caractérisé en ce que** l'affichage est un affichage électrique.

9. Système selon l'une des revendications précédentes, **caractérisé en ce que** l'on détermine, en tant qu'information sur la quantité d'éléments consommables (4) non encore utilisés en réserve sur la bande de support (3), le nombre d'éléments consommables (4) non encore utilisés.

10. Système selon l'une des revendications précédentes, **caractérisé en ce que** la vitesse de rotation du dispositif d'enroulement (5) pour le transport de la bande est réduite à mesure que le nombre d'éléments consommables (4) non encore utilisés de la bande de support (3) diminue.

11. Système selon l'une des revendications précédentes, **caractérisé par** un dispositif de commande pour régler une vitesse de déplacement de la bande sur une valeur de consigne indépendante de l'épaisseur de la bobine (6) lors de la mise à disposition d'un élément consommable (4).

12. Système selon l'une des revendications précédentes, **caractérisé en ce que** la longueur de transport de bande est détectée en fonction de la position angulaire en rotation de la bobine (6), qu'une valeur du défaut d'équilibrage de la bobine (6) est déterminée à partir de là et que l'on différencie, sur la base de cette valeur, une bande de support (3) neuve ne portant que des éléments consommables (4) non utilisés d'une bande de support (3) déjà partiellement enroulée portant au moins un élément consommable (4) utilisé.

13. Système selon l'une des revendications précédentes, **caractérisé en ce que**, lors de l'utilisation d'un élément consommable (4), une indication du compteur est modifiée de un.

14. Système selon l'une des revendications précédentes, **caractérisé en ce que** la bande de support (3) est contenue dans un boîtier de bande (1).

15. Procédé de surveillance d'une réserve restante d'éléments consommables (4) non encore utilisés qui sont portés par une bande de support (3) qui est enroulée avec les éléments consommables (4) déjà utilisés pour former une bobine (6) lors de l'utilisation, **caractérisé en ce que** l'on détermine, à partir d'un angle de rotation et d'une longueur de transport de bande sur laquelle la bande de support (3) est déplacée lors d'une rotation de la bobine (6) sur cet angle de rotation, l'information sur la quantité d'éléments consommables (4) non encore utilisés en réserve sur la bande de support (3).
